Europäisches Patentamt

European Patent Office

Office européen des brevets

(19)

(11) Veröffentlichungsnummer: **0 074 626**
**A2**

(12)  **EUROPÄISCHE PATENTANMELDUNG**

(21) Anmeldenummer: **82108321.9**

(22) Anmeldetag: **09.09.82**

(51) Int. Cl.³: **A 61 K 7/40**
**A 61 K 47/00**

(30) Priorität: **12.09.81 DE 3136308**

(43) Veröffentlichungstag der Anmeldung:
**23.03.83 Patentblatt 83/12**

(84) Benannte Vertragsstaaten:
**AT BE CH DE FR GB IT LI NL SE**

(71) Anmelder: **Meinhardt, Horst**
**Bahnhofstrasse 1**
**D-5419 Herschbach(DE)**

(72) Erfinder: **Zimzik, Henry, Dipl.-Ing.**
**Hauptstrasse 67**
**D-5419 Herschbach(DE)**

(74) Vertreter: **Goddar, Heinz, Dr. et al,**
**FORRESTER & BOEHMERT Widenmayerstrasse 4/1**
**D-8000 München 22(DE)**

(54) Verfahren zum Herstellen einer hautfreundlichen Schutzschicht.

(57) Verfahren zum Herstellen einer hautfreundlichen Schutzschicht durch Vermengung von Adeps-lanae anhydrid (Lanolin) mit Cera alba mineralis.

EP 0 074 626 A2

Croydon Printing Company Ltd

FB 481

Horst Meinhardt, Bahnhofstraße 1, D-5419 Herschbach

-------------------------------------------------------

Verfahren zum Herstellen einer hautfreundlichen Schutzschicht

-------------------------------------------------------

Die Erfindung betrifft ein Verfahren zum Herstellen
einer hautfreundlichen Schutzschicht, deren Verwendung
sowie das erfindungsgemäß hergestellte Estergemisch.

Organische Fette, insbesondere Adeps-lanae anhydrid
(Lanolin), finden als Grundlage für Kosmetika und Körperpflegemittel vielseitige Verwendung. Der Einsatz dieser
Produkte, insbesondere der von Adeps-lanae anhydrid
(Lanolin), ist jedoch durch deren Wasseraufnahmevermögen begrenzt, wo ein Ausgangsstoff zur Erzeugung von
Produkten mit hohem wasserabweisenden Effekt, guter
Haftfähigkeit und gutem Gleitvermögen bei leichter
mechanischer Beanspruchung erforderlich ist.

Der Erfindung liegt daher die Aufgabe zugrunde, durch
Beimengung eines anderen Stoffes zu dem organischen
Fett, insbesondere zu Adeps-lanae anhydrid (Lanolin) einen Ausgangsstoff zur Erzeugung von Produkten zu schaffen,
die die eben genannten Eigenschaften aufweisen.

Erfindungsgemäß wird diese Aufgabe gelöst durch die
Vermengung eines organischen Fettes mit einem Wachs,
vorzugsweise durch die Vermengung von Adeps-lanae anhydrid
(Lanolin) mit Cera alba mineralis.

Erfindungsgemäß findet diese Vermengung als Schutzüberzug für agressiven Stoffen ausgesetzte Hautpartien, aber
auch als Grundlage für Salben und für sonstige kosmetische oder
pharmazeutische Zwecke Verwendung.

Es hat sich gezeigt, daß durch die Beimengung von Wachs,
insbesondere von Cera alba mineralis,    zu einem orgasichen Fett, insbesondere Adeps-lanae anhydrid (Lanolin), ein
knetbares, kaum fettendes, elastisches Gemisch erhalten
wird, dessen Eigenschaften, insbesondere dessen Wasseraufnahmefähigkeit, durch das Verhältnis der Ausgansstoffe
bestimmt ist.

Das so erhaltene Produkt weist die bekannten hautfreundlichen Eigenschaften der beiden Ausgangsprodukten auf,
Lösungen des erfindungsgemäßen Gemisches haften gut auf
der menschlichen Haut, ohne deren Atmungsaktivität zu
beeinträchtigen.

So wurde eine Vermengung aus Adeps-lanae anhydrid (Lanolin) und
Cera alba mineralis    in einer alkoholischen Lösung
(Isopropylalkohol) als Industriehandschuh in der metallbehandelnden Industrie verwendet. Dabei zeigte sich, daß berufsbedingte Hautschäden nach kurzer Zeit abheilten.
Die Versuchspersonen bekundeten, daß der Umgang mit
Metallen durch die Schutzschicht nicht nachteilig beeinflußt worden ist, insbesondere litten weder der
Tastsinn noch das Wärme- oder Kälteempfinden an den
Händen. Auch ständiges Arbeiten unter fließendem oder
in stehendem Wasser war möglich, ohne daß die bekannten
Hautschädigungen festgestellt wurden.

Weiter wurde festgestellt, daß das erfindungsgemäße Produkt
haltbar ist, und das Fertigprodukt weder oxidativ noch
bakteriell anfällig ist oder verändert wird. Für
die Verwendung als hautfreundliche Schutzschicht im
industriellen Bereich hat sich eine Zusammenschmelzung
von Adeps-lanae anhydrid mit 5 bis 20 % Cera alba mineralis als besonders
vorteilhaft erwiesen. Dieses Produkt weist hervorragend
Schmutz ab, es bildet weiter eine Kontaminierungssperre
zwischen der menschlichen Haut und beispielsweise waschaktiven Substanzen. So können Personen, die an Waschmittelallergien leiden, unbedenklich mit Waschmitteln
umgehen, wenn die Hände zuvor mit einer alkoholischen
Lösung des Gemisches eingesprüht werden.

Es hat sich gezeigt, daß Hautverträglichkeit und Belastbarkeit der Schutzschicht dann besonders günstig ist,
wenn sie mit Milchsäure auf einen PH-Wert von 5 bis 4
angesäuert wird.

Eine chemische Analyse des erfindungsgemäßen Gemisches
zeigt, daß dieses Cerotinsäure, Ölsäure, Capronsäure
und Palmitinsäure ($C_{30}H_{60}O_4$) als Fettsäuren, Myristinsäure und Lanopalminsäure ($C_{16}H_{32}O_3$), freie Milchsäure
($C_3H_6O_3$), und Isocholesterin, Cholesterin, Cetylalkohol, Cerylalkohol, Lanolinalkohol ($C_{12}H_{24}O$), Carnaubyl-

alkohol ($C_{24} H_{50} O$) als Alkohole und hochmolekulare
Paraffin-KW-Stoffe (etwa von $C_{18} H_{33}$ an aufwärts) und
geringen Anteilen an harzartigen, sauerstoffhaltigen
Stoffen, aufweist.

Die in der vorstehenden Beschreibung sowie in den Ansprüchen offenbarten Merkmale der Erfindung können
sowohl einzeln als auch in beliebiger Kombination für
die Verwirklichung der Erfindung in ihren verschiedenen
Ausführungsformen wesentlich sein.

0074626

Ansprüche
==================

1. Verfahren zum Herstellen einer hautfreundlichen Schutzschicht, gekennzeichnet durch die Vermengung eines organischen Fettes mit einem Wachs.

2. Verfahren nach Anspruch 1, gekennzeichnet durch die Verwendung von Adeps-lanae anhydrid (Lanolin) als organisches Fett.

3. Verfahren nach Anspruch 1 oder 2, gekennzeichnet durch
die Verwendung von Cera alba mineralis als Wachs.

4. Verfahren nach einem der vorangehenden Ansprüche, gekennzeichent durch die Anlagerung einer Säure an das organische
Fett vor der Vermengung der Ausgangsstoffe.

5. Verfahren nach Anspruch 4, gekennzeichnet durch die Verwendung von Milchsäure als angelagerte Säure.

6. Verwendung einer Vermengung aus organischem Fett, vorzugsweise Adeps-lanae anhydrid, mit einem Wachs, vorzugsweise Cera alba mineralis, als Schutzüberzug für agressiven
Stoffen ausgesetzte Hautpartien.

7. Verwendung einer Vermengung aus organischem Fett, vorzugsweise Adeps-lanae anhydrid, mit einem Wachs, vorzugsweise
Cera alba mineralis, als Grundlage für Salben oder für sonstige kosmetische
oder pharmazeutische Zwecke.

925

**0074626**

8. Estergemisch mit Cerotinsäure, Ölsäure, Capronsäure und Palmitinsäure ($C_{30} H_{60} O_4$) als Fettsäuren, Myristinsäure und Lanopalminsäure ($C_{16} H_{32} O_3$), freie Milchsäure ($C_3 H_6 O_3$), und Isocholesterin, Cholesterin, Cetylalkohol, Ceryl-alkohol, Lanolinalkohol ($C_{12} H_{24} O$), Carnaubyalkohol ($C_{24} H_{50} O$) als Alkohole und hochmolekulare Paraffin-KW-Stoffe (etwa von $C_{18} H_{33}$ an aufwärts) und geringen Anteilen an harzartigen, sauerstoffhaltigen Stoffen.